(19)

Europäisches Patentamt
European Patent Office
Office européen des brevets

(11) **EP 4 290 613 A1**

(12) **EUROPEAN PATENT APPLICATION**
published in accordance with Art. 153(4) EPC

(43) Date of publication:
**13.12.2023 Bulletin 2023/50**

(21) Application number: **22776046.9**

(22) Date of filing: **22.03.2022**

(51) International Patent Classification (IPC):
*H01M 4/525* (2010.01)        *H01M 4/505* (2010.01)
*H01M 4/36* (2006.01)         *H01M 4/62* (2006.01)
*H01M 10/052* (2010.01)       *H01M 4/02* (2006.01)

(52) Cooperative Patent Classification (CPC):
**H01M 4/02; H01M 4/36; H01M 4/505; H01M 4/525;
H01M 4/62; H01M 10/052;** Y02E 60/10

(86) International application number:
**PCT/KR2022/003972**

(87) International publication number:
**WO 2022/203346 (29.09.2022 Gazette 2022/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.03.2021 KR 20210036940**

(71) Applicant: **Lg Chem, Ltd.**
**Seoul 07336 (KR)**

(72) Inventors:
 • **JUNG, Won Sig**
  **Daejeon 34122 (KR)**

 • **CHOI, Hwan Young**
  **Daejeon 34122 (KR)**
 • **PARK, Hyun Ah**
  **Daejeon 34122 (KR)**
 • **BAEK, Hyeon Hui**
  **Daejeon 34122 (KR)**
 • **KIM, Jong Pil**
  **Daejeon 34122 (KR)**

(74) Representative: **Hoffmann Eitle
Patent- und Rechtsanwälte PartmbB
Arabellastraße 30
81925 München (DE)**

(54) **CATHODE ACTIVE MATERIAL, CATHODE COMPRISING SAME, AND LITHIUM SECONDARY BATTERY**

(57)    The present invention relates to a cathode active material, a cathode comprising same, and a lithium secondary battery, wherein crystal grains A account for 25% to 80% of all the crystal grains on the cathode active material particle cross-section, the crystal grains A having: a crystal grain major axis orientation DoA of 0.5-1; and a crystal grain c-axis orientation of 0.5-1 as expressed by an outer product of the c-axis rotation vector Rc of the crystal lattice of a crystal grain and the position unit vector P' of the crystal grain, obtained through electron backscatter diffraction (EBSD) analysis.

[FIG. 3]

EP 4 290 613 A1

**Description**

## TECHNICAL FIELD

This application claims the benefit of Korean Patent Application No. 10-2021-0036940, filed on March 22, 2021, the disclosure of which is incorporated herein in its entirety by reference.

The present invention relates to a positive electrode active material, a positive electrode including the same, and a lithium secondary battery, and more particularly, to a positive electrode active material for a lithium secondary battery having excellent life characteristics and resistance characteristics, a positive electrode including the same, and a lithium secondary battery.

## BACKGROUND ART

Demand for secondary batteries as an energy source has been significantly increased as technology development and demand with respect to mobile devices and electric vehicles have recently increased. Among these secondary batteries, lithium secondary batteries having high energy density, high voltage, long cycle life, and low self-discharging rate have been commercialized and widely used.

Lithium transition metal oxides, such as lithium cobalt oxide such as $LiCoO_2$, lithium nickel oxide such as $LiNiO_2$, lithium manganese oxide such as $LiMnO_2$ or $LiMn_2O_4$, or lithium iron phosphate such as $LiFePO_4$, have been developed as a positive electrode active material of the lithium secondary battery, and, recently, lithium composite transition metal oxides including two or more types of transition metals, for example, $Li[Ni_aCo_bMn_c]O_2$, $Li[Ni_aCo_bAl_c]O_2$, and $Li[Ni_aCo_bMn_cAl_d]O_2$, have been developed and widely used.

Lithium composite transition metal oxides including two or more transition metals which have been developed so far are generally prepared in the form of spherical secondary particles in which dozens to hundreds of primary particles are aggregated. Physical properties such as mobility of lithium ions or impregnation of an electrolyte may vary depending on the orientation form of the primary particles or the shape (aspect ratio) of the primary particles. Accordingly, studies to improve the performance of positive electrode active materials by controlling the particle structure of positive electrode active material particles have been attempted.

Korean Patent No. 10-1611784 (Patent Document 1) discloses a positive electrode active material in which the length of an a-axis direction of a primary particle is longer than the length of a c-axis direction, and the a-axis of the primary particle is radially arranged. In Patent Document 1, the shape of the primary particles or the orientation of the primary particles of the positive electrode active material was analyzed using a scanning electron microscope (SEM) and/or a transmission electron microscope (TEM).

However, the TEM analysis used in Patent Document 1 has a problem in that it is difficult to represent the properties of the entire positive electrode active material particles because only information on a partial region rather than the entire particles may be obtained. In addition, since the physical properties of the positive electrode active material vary depending on the shape or orientation of the crystallites as well as the shape or orientation of the primary particles, the positive electrode active material may exhibit different physical properties even when the shape or orientation of the primary particles is similar.

Therefore, in order to develop a positive electrode active material having better properties, studies on a crystallite structure of the positive electrode active material are being required.

## DISCLOSURE OF THE INVENTION

## TECHNICAL PROBLEM

An aspect of the present invention provides a positive electrode active material which can achieve excellent capacity characteristics and service life characteristics by including, in a particular proportion, crystallites in which the orientation of long-axis and c-axis of the crystallite satisfies a particular condition.

Another aspect of the present invention provides a positive electrode and a lithium secondary battery which include the positive electrode active material according to the present invention.

## TECHNICAL SOLUTION

According to an aspect of the present invention, there is provided a positive electrode active material for a lithium secondary battery in which the proportion of crystallites A, in which a crystallite long-axis orientation degree DoA represented by Equation 1 below is 0.5 to 1 and a crystallite c-axis orientation degree represented by an cross product value of a c-axis rotation vector Rc of a crystal lattice of a crystallite obtained by electron backscatter diffraction (EBSD) analysis and a position unit vector P' of the crystallite is 0.5 to 1, is 25% to 80% with respect to total crystallites in the cross-section of a positive electrode active material particle.

$$[\text{Equation 1}]$$

$$DoA = \frac{\lambda_1}{\lambda_1 + \lambda_2} C_D{}^2$$

In Equation 1 above, $\lambda_1$ is the size of a long-axis vector $E_I$ of the crystallite determined from image data obtained by scanning ion microscope analysis of the cross-section of the positive electrode active material, $\lambda_2$ is the size of a short-axis vector $E_{II}$ of the crystallite determined from image data obtained by scanning ion microscope analysis of the cross-section of the positive electrode active material, and $C_D$ above is an inner product value of a position unit vector P' and a long-axis vector $E_I{}'$ of the crystallite.

In this case, the scanning ion microscope analysis may be performed by obtaining a scanning ion microscope image by irradiating the cross-section of the positive electrode active material with a focused ion beam, then obtaining data segmented in a crystallite unit from the scanning ion microscope image using deep learning, and calculating DoA represented by Equation 1 from the segmented data.

Meanwhile, the EBSD analysis may be performed by obtaining EBSD Euler map data including position information and Euler angle information on each crystallite through EBSD measurement of the cross-section of the positive electrode active material, and calculating the c-axis rotation vector Rc (x, y, z) of the crystal lattice of the crystallite by means of Equation 2 below:

$$[\text{Equation 2}]$$

$$\begin{bmatrix} x \\ y \\ z \end{bmatrix} = R_z(\Psi)R_y(\Theta)R_x(\Phi) \begin{bmatrix} X \\ Y \\ Z \end{bmatrix}$$

$$= \begin{bmatrix} \cos\Psi & -\sin\Psi & 0 \\ \sin\Psi & \cos\Psi & 0 \\ 0 & 0 & 1 \end{bmatrix} \begin{bmatrix} \cos\Theta & 0 & \sin\Theta \\ 0 & 1 & 0 \\ -\sin\Theta & 0 & \cos\Theta \end{bmatrix} \begin{bmatrix} 1 & 0 & 0 \\ 0 & \cos\Phi & -\sin\Phi \\ 0 & \sin\Phi & \cos\Phi \end{bmatrix} \begin{bmatrix} X \\ Y \\ Z \end{bmatrix}$$

$$= \begin{bmatrix} \cos\Theta\cos\Psi & -\cos\Phi\sin\Psi+\sin\Phi\sin\Theta\cos\Psi & \sin\Phi\sin\Psi+\cos\Phi\sin\Theta\cos\Psi \\ \cos\Theta\sin\Psi & \cos\Phi\cos\Psi+\sin\Phi\sin\Theta\sin\Psi & -\sin\Phi\cos\Psi+\cos\Phi\sin\Theta\sin\Psi \\ -\sin\Theta & \sin\Phi\cos\Theta & \cos\Phi\cos\Theta \end{bmatrix} \begin{bmatrix} X \\ Y \\ Z \end{bmatrix}$$

In Equation 2 above,
[X, Y, Z] is (0, 0, 1), and $\Phi$, $\theta$, and $\Psi$ above each represent Euler angle obtained from Euler map data.

Preferably, the positive electrode active material may further include crystallites B in which the crystallite c-axis orientation degree is 0.5 to 1 and the DoA is less than 0.5, crystallites C in which the crystallite c-axis orientation degree is less than 0.5 and the DoA is 0.5 to 1, and crystallites D in which the crystallite c-axis orientation degree is less than 0.5 and the DoA is less than 0.5.

In this case, the proportion of the sum of the crystallites A and the crystallites C in the total crystallites in the cross-section of the positive electrode active material particle may be 50% to 90%, preferably 50% to 80%, and specifically, the proportion of the crystallites A in the total crystallites in the cross-section of the positive electrode active material particle may be 25% to 70%, the proportion of the crystallites B may be 5% to 30%, the proportion of the crystallites C may be 20% to 70%, and the proportion of the crystallites D may be 5% to 30%.

Meanwhile, the positive electrode active material may have a crystallite size of 100 nm to 200 nm, preferably 100 nm to 180 nm, and more preferably, 100 nm to 150 nm.

In addition, the positive electrode active material may have a micro strain of 0.04% to 0.25%, and preferably, 0.06% to 0.15%.

In addition, the positive electrode active material may have an average particle diameter of primary particles

of 0.05 $\mu$m to 8 $\mu$m, preferably, 0.1 $\mu$m to 4 $\mu$m, and an average particle diameter of secondary particles of 2 $\mu$m to 25 pm, preferably, 4 $\mu$m to 18 $\mu$m.

Meanwhile, the positive electrode active material may be a lithium composite transition metal oxide represented by Formula 1 below.

$$[\text{Formula 1}] \qquad Li_x[Ni_aCo_bM^1_cM^2]O_{2-y}A_y$$

In Formula 1 above,

$M^1$ above is at least one element selected from the group consisting of Mn and Al, $M^2$ above is at least one element selected from the group consisting of W, Cu, Fe, V, Cr, Ti, Zr, Zn, Al, Ta, Y, In, La, Sr, Ga, Sc, Gd, Sm, Ca, Ce, Nb, Mg, B, and Mo, and A above is at least one element selected from the group consisting of F, Cl, Br, I, At, and S, and $0.98 \le x \le 1.20$, $0 < a < 1$, $0 < b < 1$, $0 < c < 1$, $0 \le d \le 0.2$, $0 \le y \le 0.2$.

According to another aspect of the present invention, there is provided a positive electrode including the positive electrode active material according to the present invention, and a lithium secondary battery including the positive electrode.

## ADVANTAGEOUS EFFECTS

The positive electrode active material of the present invention includes, in a particular proportion, crystallites having high long-axis orientation and c-axis orientation thereof, and thus may achieve excellent capacity characteristics and service life characteristics when applied to a secondary battery.

## BRIEF DESCRIPTION OF THE DRAWINGS

FIG. 1 is a view showing a scanning ion microscope image of the cross-section of a positive electrode active material;
FIG. 2 is a view showing a process of obtaining a segmentation image by analyzing a scanning ion microscope image of the cross-section of a positive electrode active material;
FIG. 3 is a view showing long-axis orientation and DoA values of crystallites;
FIG. 4 is a view showing an electron backscatter diffraction (EBSD) Euler map obtained by performing EBSD analysis on the cross-section of the positive electrode active material; and
FIG. 5 is a view showing a c-axis orientation map of crystallites.

## BEST MODE FOR CARRYING OUT THE INVENTION

It will be understood that words or terms used in the specification and claims shall not be interpreted as the meaning defined in commonly used dictionaries, and it will be further understood that the words or terms should be interpreted as having a meaning that is consistent with their meaning in the context of the relevant art and the technical idea of the invention, based on the principle that an inventor may properly define the meaning of the words or terms to best explain the invention.

In the present invention, the term "crystallite" refers to a single crystal particle unit having a regular atom arrangement. In the present invention, the size of the crystallite may be measured by analyzing, in the Rietveld refinement method, X-ray diffraction data of the cross-sections of positive electrode active materials. For example, the crystallite size may be obtained by performing X-ray diffraction analysis under the following conditions using Empyreon XRD equipment from Malyer Panalytical, Ltd. to obtain XRD data, and then processing the XRD data using Highscore program from Malyer Panalytical, Ltd. In this case, a full width at half maximum is set to be measured using Caglioti equation.

<X-ray diffraction analysis conditions>

Light source: Cu-target, 45 kV, 40 mA output, wavelength=1.54 Å
Detector: GaliPIX3D
Sample preparation: About 5 g of a sample is filled in a holder having a diameter of 2 cm and loaded on a rotation stage.
Measurement time: about 30 minutes
Measurement region: $2\theta$=15° to 85°

In the present invention, the term "primary particle" refers to a minimum particle unit distinguished as a single lump when the cross-section of the positive electrode active material is observed through a scanning electron microscope (SEM), and may be composed of a single crystallite or a plurality of crystallites. In the present invention, an average particle diameter of the primary particles may be determined by a method of measuring each particle size distinguished from cross-sectional SEM data of the positive electrode active material.

In the present invention, the term "secondary particle" refers to a secondary structure formed by agglomerating a plurality of primary particles. The average particle diameter of the secondary particles may be measured by using a particle size analyzer, and in the present invention, s3500 manufactured by Microtrac is used as a particle size analyzer.

In the present invention, the term "micro strain" refers to a value measured through Rietveld refinement analysis of X-ray diffraction data, which shows a degree of deformation in a crystal lattice .

Meanwhile, in the present invention, the proportion (%) of each crystallite means (the number of the crystallites / the number of the total crystallites present on the cross-section of the positive electrode active material particles) $\times$ 100.

Hereinafter, the present invention will be described in detail.

As a result of a significant amount of research conducted for developing a positive electrode active material capable of achieving excellent service life characteristics and resistance characteristics, the present inventors have found that when the proportion of crystallites having high long-axis and c-axis orientation thereof among crystallites of the positive electrode active material satisfies a particular range, capacity characteristics and service life characteristics of a secondary battery may be improved, thereby leading to the completion of the present invention.

**Positive Electrode Active Material**

The positive electrode active material according to the present invention is characterized in that the proportion of crystallites A, in which a crystallite long-axis orientation degree DoA represented by Equation 1 is 0.5 to 1 and a crystallite c-axis orientation degree is 0.5 to 1, satisfies 25% to 80% in the total crystallites in the cross-sections of positive electrode active material particles.

$$[\text{Equation 1}]$$

$$DoA = \frac{\lambda_1}{\lambda_1 + \lambda_2} C_D{}^2$$

In Equation 1 above,

$\lambda_1$ is the size of a long-axis vector $E_l$ of the crystallite determined from image data obtained by scanning ion microscope analysis of the cross-section of the positive electrode active material, $\lambda_2$ is the size of a short-axis vector $E_{ll}$ of the crystallite determined from image data obtained by scanning ion microscope analysis of the cross-section of the positive electrode active material, and $C_D$ above is an inner product value of a position unit vector P' and a long-axis vector $E_l$' of the crystallite.

First, the DoA represented by Equation 1 above will be described.

The DoA value represented by Equation 1 above is to show a long-axis orientation of the crystallites, and can be determined by using data obtained through a scanning ion microscope analysis.

Specifically, a scanning ion microscope image may be obtained by irradiating the cross-section of the positive electrode active material with a focused ion beam, data segmented in a crystallite unit may be obtained from the scanning ion microscope image using deep learning, and a crystallite long-axis orientation degree DoA represented by Equation 1 may be calculated from the segmented data.

Hereinafter, a method for calculating a DoA value through a scanning ion microscope analysis will be described in more detail.

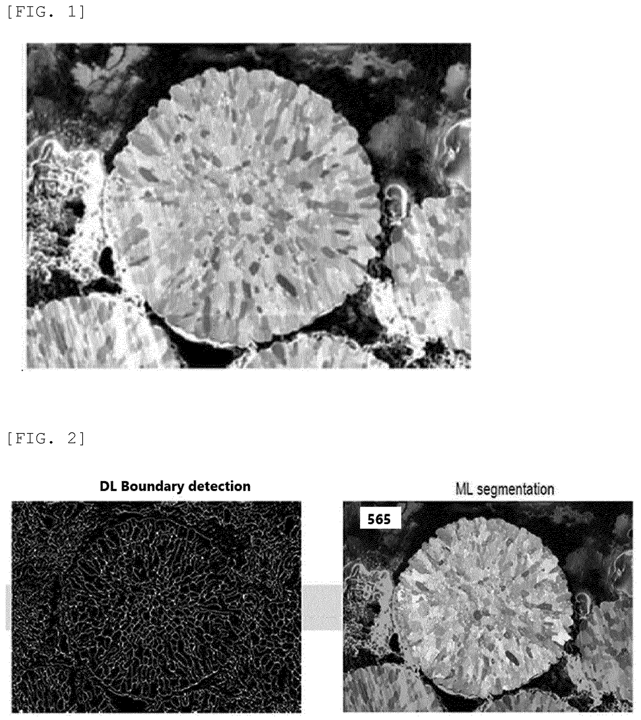

A scanning ion microscope is a device for measuring the surface structure of a sample through the signal ion images generated when scanning the ion beam on the surface of the sample. In this case, since the ion beam has different reflectivity on different crystalline surfaces, the cross-sectional image of the positive electrode active material particle distinguished in a unit of a single crystallite having the same atomic arrangement can be obtained using the scanning ion microscope. FIG. 1 shows a scanning ion microscope image of the cross-section of a positive electrode active material particle. Through FIG. 1, it can be confirmed that the cross-sectional image of the positive electrode active material particle is distinguished in a crystallite unit.

Next, the scanning ion microscope image thus obtained is analyzed to obtain data segmented in a crystallite unit. In this case, the image analysis may be performed using deep learning.

FIG. 2 shows a process of obtaining segmented data information by analyzing the scanning ion microscope image. As shown in FIG. 2, the image analysis may be performed by, for example, detecting a boundary from the scanning ion microscope image through deep learning and then obtaining image data segmented in a crystallite unit using the boundary.

In this case, the boundary detection may be performed using an AutoEncoder neural network (U-NET) algorithm, and the segmentation may be performed using a Watershed segmentation algorithm or the like.

Since the scanning ion microscope image itself does not include digitized information, in the present invention, data information segmented in each crystallite unit may be obtained through deep learning, and thus information such as the shape and position of the crystallites may be digitized.

When the segmented data are obtained through the scanning ion microscope image analysis, the position vector, long-axis vector, and short-axis vector of the crystallites to be determined may be calculated from the data, and using this, a DoA value in Equation 1 may be calculated.

[Equation 1]

$$DoA = \frac{\lambda_1}{\lambda_1 + \lambda_2} C_D{}^2$$

In Equation 1 above,

$\lambda_1$ is the size of a long-axis vector $E_l$ of the crystallite determined from image data obtained by the scanning ion microscope analysis of the cross-section of the positive electrode active material, wherein the long-axis vector $E_l$ means a vector having the smallest sum of distances between the vector and each pixel in the crystallite among vectors passing through the center of gravity of the crystallite.

$\lambda_2$ is the size of a short-axis vector $E_{ll}$ of the crystallite determined from image data obtained by the scanning ion microscope analysis of the cross-section of the positive electrode active material, wherein the short-axis vector $E_{ll}$ means a vector having the largest sum of distances between a single vector and each pixel in the crystallite among vectors passing through the center of gravity of the crystallite.

Meanwhile, $C_D$ above is an inner product value of a position unit vector P' and the long-axis vector $E_l'$ of the crystallite, the position unit vector P' of the crystallite is a vector obtained by converting the position vector, which connects from the center of the cross-section of the positive electrode active material particle to the center of gravity of the crystallite, into a size of 1, and the long-axis unit vector $E_l'$ is a vector obtained by converting the long-axis vector $E_l$ into a size of 1.

The DoA value calculated by Equation 1 is a value showing how much the long-axis of the crystallite is inclined with respect to a straight line passing through the center of the positive electrode active material and the center of gravity of the crystallite, and means that the closer to 1 the DoA value, the smaller the angle between the long-axis of the crystallite and the straight line, and the closer to 0 the DoA value, the larger the angle between the long-axis of the crystallite and the straight line. That is, it can be said that the closer to 1 the DoA, the higher the long-axis orientation of the crystallite.

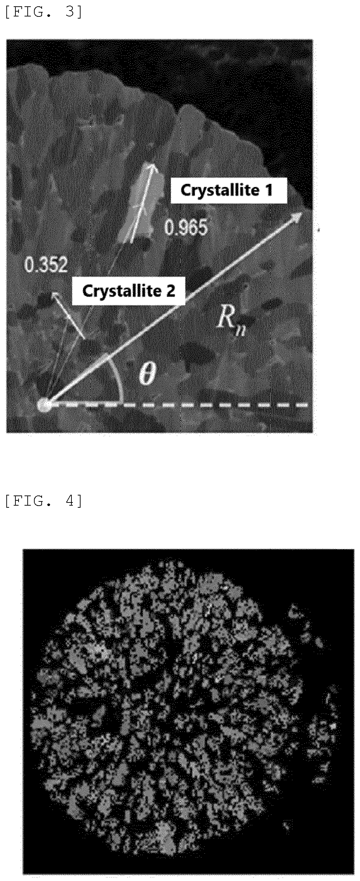

FIG. 3 shows a view in which the DoA value obtained by the method and the long-axis of the crystallite are indicated. As shown in FIG. 3, it can be seen that in the case of a crystallite 1 having a small angle between the crystallite long-axis and a straight line passing through the center of the positive electrode active material and the center of gravity of the crystallite, DoA is 0.965, which is close to 1, whereas a crystallite 2 having a large angle between the crystallite long-axis and a straight line passing through the center of the positive electrode active material and the center of gravity of the crystallite, DoA is 0.352, which is small.

Meanwhile, the proportion of the crystallites having a specific long-axis orientation degree value in the cross-section of the positive electrode active material particle may be measured by mapping the long-axis orientation degree information of each crystallite as described above.

Next, the crystallite c-axis orientation degree will be described.

The crystallite c-axis orientation degree is to show the orientation of a c-axis of a crystal lattice of a crystallite, and an cross product value of a c-axis rotation vector Rc of the crystal lattice of the crystallite obtained by electron backscatter diffraction (EBSD) analysis and a position unit vector P' of the crystallite.

Specifically, the crystallite c-axis orientation degree may be determined by obtaining EBSD Euler map data including position information and Euler angle information on each crystallite through EBSD measurement of the cross-section of the positive electrode active material, calculating the c-axis rotation vector Rc of the crystal lattice of the crystallite using the EBSD Euler map data, and calculating the cross product of the c-axis rotation vector Rc of the crystal lattice and the position unit vector P' of the crystallite.

Hereinafter, a method for determining the crystallite c-axis orientation degree according to the present invention will be described in detail.

The EBSD analysis is a method for measuring a crystallographic phase and a crystallographic orientation using a diffraction pattern of a sample and analyzing crystallographic information of the sample based on them. In the scanning electron microscope, if the sample (i.e., the cross-section of the positive electrode active material) is tilted to have a large angle with respect to the incident direction of the electron beam, the incident electron beam is scattered in the sample, and thus a diffraction pattern appears in the direction of the surface of the sample, which is called Electron Back Scattered Diffraction Pattern (EBSP). Since the EBSP responds to the crystallographic orientation of the region irradiated with the electron beam, the crystallographic orientation of the sample may be accurately measured by using the EBSP, and Euler map data including various information related to the crystallographic orientation of the entire sample may be obtained through an EBSD software. FIG. 4 shows a Euler map obtained by performing the EBSD analysis on the cross-section of the positive electrode active material particle.

The EBSD Euler map data includes position vector information and Euler angle information of each crystallite. Meanwhile, by using the Euler angle information, the c-axis rotation vector Rc of the crystal lattice in each crystallite may be obtained.

The c-axis rotation vector Rc of the crystal lattice shows what direction the c-axis of the crystallite is rotated with respect to a straight line passing through the center of gravity of the crystallite and the center of the positive electrode active material.

Specifically, the c-axis rotation vector Rc of the crystal lattice may be (x, y, z) calculated by Equation 2 below:

[Equation 2]

$$
\begin{bmatrix} x \\ y \\ z \end{bmatrix} = R_z(\Psi)R_y(\Theta)R_x(\Phi) \begin{bmatrix} X \\ Y \\ Z \end{bmatrix}
$$

$$
= \begin{bmatrix} \cos\Psi & -\sin\Psi & 0 \\ \sin\Psi & \cos\Psi & 0 \\ 0 & 0 & 1 \end{bmatrix} \begin{bmatrix} \cos\Theta & 0 & \sin\Theta \\ 0 & 1 & 0 \\ -\sin\Theta & 0 & \cos\Theta \end{bmatrix} \begin{bmatrix} 1 & 0 & 0 \\ 0 & \cos\Phi & -\sin\Phi \\ 0 & \sin\Phi & \cos\Phi \end{bmatrix} \begin{bmatrix} X \\ Y \\ Z \end{bmatrix}
$$

$$
= \begin{bmatrix} \cos\Theta\cos\Psi & -\cos\Phi\sin\Psi+\sin\Phi\sin\Theta\cos\Psi & \sin\Phi\sin\Psi+\cos\Phi\sin\Theta\cos\Psi \\ \cos\Theta\sin\Psi & \cos\Phi\cos\Psi+\sin\Phi\sin\Theta\sin\Psi & -\sin\Phi\cos\Psi+\cos\Phi\sin\Theta\sin\Psi \\ -\sin\Theta & \sin\Phi\cos\Theta & \cos\Phi\cos\Theta \end{bmatrix} \begin{bmatrix} X \\ Y \\ Z \end{bmatrix}
$$

In Equation 2 above, [X, Y, Z] is (0, 0, 1), and $\Phi$, $\theta$, and $\Psi$ above each represent Euler angle of each crystallite obtained from the Euler map data.

A crystallite orientation degree may be obtained by using the c-axis rotation vector Rc of the crystal lattice as obtained above and the position vector information of each crystallite included in the Euler map data. Specifically, the crystallite orientation degree may be digitized as a value obtained by calculating an cross product of the c-axis rotation vector Rc of the crystal lattice and the position unit vector P' of the crystallite.

In this case, the position unit vector P' means that the position vector of the crystallite is converted to have a size of 1. For example, if the position vector of the crystallite is (a, b, o), the position unit vector becomes $(\frac{a}{\sqrt{a^2+b^2}}, \frac{b}{\sqrt{a^2+b^2}}, 0)$.

An inner product value of position unit vector P' and the c-axis rotation vector Rc of the crystal lattice is a numerical value showing the c-axis orientation degree of the crystallite in the positive electrode active material particle. Specifically, when the cross product value of the position unit vector P' and the c-axis rotation vector Rc of the crystal lattice is 1, it means that the c-axis of the crystallite is disposed perpendicular to a straight line passing through the center of the positive electrode active material particle and the center of gravity of the crystallite, and when the cross product value is 0, it means that the c-axis of the crystallite is disposed parallel to the straight line.

In the positive electrode active material, the mobility of lithium ions when moving along the direction perpendicular to the c-axis is at least 10 times faster than when moving in the c-axis direction. Accordingly, a lithium path is formed along the direction perpendicular to the c-axis. In addition, when the lithium path is formed parallel to the straight line passing through the center of the positive electrode active material particle and the center of gravity of the crystallite, a lithium moving distance is minimized, and thus lithium conductivity is improved. Therefore, it may be determined that the c-axis orientation of the crystallite is excellent as the cross product value of the position unit vector P' and the c-axis rotation vector Rc of the crystal lattice is closer to 1.

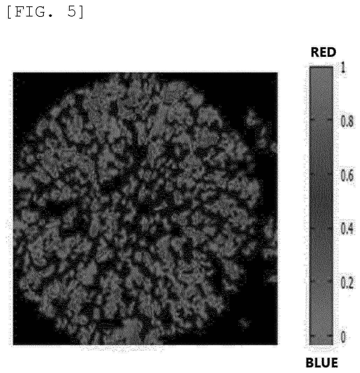

Meanwhile, by collecting the c-axis orientation degree of each crystallite as obtained above, the c-axis orientation degree of the total crystallites in the cross-section of the positive electrode active material particle may be obtained. FIG. 5 shows a crystallite c-axis orientation map of the positive electrode active material obtained by collecting the c-axis orientation degree of each crystallite. In FIG. 5, it means that the c-axis orientation becomes superior as it goes closer to red, and the c-axis orientation becomes inferior as it goes closer to blue. By using the c-axis orientation map as described above, the proportion of the crystallites satisfying the c-axis orientation condition in the cross-section of the positive electrode active material particle may be obtained.

According to the study of the present inventors, the proportion of the crystallites, in which the crystallite long-axis orientation degree DoA represented by Equation 1 is 0.5 to 1 and the crystallite c-axis orientation degree is 0.5 to 1, (hereinafter, referred to as crystallites A) in the total crystallites in the cross-section of the positive electrode active material particle may be 25% to 80%, 25% to 70%, 30% to 70%, 30% to 60%, 30% to 50%, or 30% to 40%. When the proportion of the crystallites A satisfies the above range, it was found that excellent service life characteristics and capacity characteristics may be achieved. When the proportion of the crystallites A is less than 25%, the effect of improving service life characteristics and resistance characteristics cannot be obtained, and when the proportion of the crystallites A is greater than 80%, capacity characteristics are degraded.

Meanwhile, in addition to the crystallites A, the positive electrode active material according to the present invention may further include crystallites having a crystallite c-axis orientation degree of 0.5 to 1 and a crystallite long-axis orientation degree DoA of less than 0.5 (hereinafter, referred to as crystallites B), crystallites having a crystallite c-axis orientation degree of less than 0.5 and a crystallite long-axis orientation degree DoA of 0.5 to 1 (hereinafter, referred to as crystallites C), and crystallites having a crystallite c-axis orientation degree of less than 0.5 and a crystallite long-axis orientation degree DoA of less than 0.5 (hereinafter, referred to as crystallites D) .

In this case, it is preferable that the proportion of the sum of the crystallites A and the crystallites C in the total crystallites in the cross-section of the positive electrode active material particle is 50% to 90%, specifically 50% to 80%, and more specifically 55% to 65%. When the proportion of the crystallites A and the crystallites C satisfies the above range, a superior effect may be obtained in terms of service life characteristics, particularly resistance characteristics.

Specifically, the proportion of the crystallites A in the total crystallites in the cross-section of the positive electrode active material particle may be 25% to 70%, preferably 30% to 60%, more preferably 30% to 50%, and even more preferably 30% to 50%, the proportion of the crystallites B may be 5% to 30%, preferably 10% to 30%, more preferably 15% to 30%, and even more preferably 15% to 25%, the proportion of the crystallites C may be 20% to 70%, preferably 20% to 50%, and more preferably 20% to 40%, and the proportion of the crystallites D may be 5% to 30%, preferably 10% to 30%, more preferably 15% to 30%, and even more preferably 10% to 20%.

Meanwhile, since the proportion of the crystallites of the positive electrode active material varies depending on the composition of a precursor, the shape and orientation of the crystallite of the precursor, the type of doping elements, and/or firing temperature, etc., the positive electrode active material satisfying the proportion of the crystallites of the present invention may be prepared by appropriately adjusting the type of precursors, the doping elements, the firing temperature, etc.

Meanwhile, the positive electrode active material according to the present invention may be a lithium composite transition metal oxide including two or more transition metals, for example, may be a lithium composite transition metal oxide represented by Formula 1 below:

$$[\text{Formula 1}] \qquad Li_x[Ni_aCo_bM^1_cM^2_d]O_{2-y}A_y$$

In Formula 1, $M^1$ above may be at least one element selected from the group consisting of Mn and Al.

$M^2$ above may be at least one element selected from the group consisting of W, Cu, Fe, V, Cr, Ti, Zr, Zn, Al, Ta, Y, In, La, Sr, Ga, Sc, Gd, Sm, Ca, Ce, Nb, Mg, B, and Mo.

In addition, A above may be at least one element selected from the group consisting of F, Cl, Br, I, At, and S.

x above represents a ratio of the moles of Li to the total moles of transition metals, wherein x may satisfy $0.98 \leq x \leq 1.20$, preferably $0.99 \leq x \leq 1.10$, and more preferably $1.0 \leq x \leq 1.10$.

a above represents a ratio of the moles of Ni to the total moles of transition metals, wherein a may satisfy $0 < a < 1$, preferably $0.3 \leq a < 1$, more preferably $0.6 \leq a < 1$, and even more preferably $0.8 \leq a < 1$.

b above represents a ratio of the moles of Co to the total moles of transition metals, wherein b may satisfy $0 < b < 1$, preferably $0 < b < 0.7$, more preferably $0 < b < 0.4$, and even more preferably $0 < b < 0.2$.

c above represents a ratio of the moles of $M^1$ to the total moles of transition metals, wherein c may satisfy $0<c<1$, preferably $0<c<0.7$, more preferably $0<c<0.4$, and even more preferably $0<c<0.2$.

d above represents a ratio of the moles of $M^2$ to the total moles of transition metals, wherein d may satisfy $0\leq d\leq 0.2$, preferably $0\leq d\leq 0.15$, and more preferably $0\leq d\leq 0.10$.

y above represents a ratio of the moles of element A substituted at the position of oxygen, wherein y may satisfy $0\leq y\leq 0.2$, preferably $0\leq y\leq 0.15$, and more preferably $0\leq y\leq 0.10$.

Meanwhile, the positive electrode active material may have a crystallite size of 100 nm to 200 nm, preferably 100 nm to 180 nm, and more preferably 100 nm to 150 nm. If the size of the crystallite becomes too large, a rock salt phase may be formed, and thus resistance characteristics and service life characteristics may be degraded, and if the size of the crystallite becomes too small, a contact area with an electrolyte may increase, and thus the deterioration may occur rapidly.

In addition, the positive electrode active material may have a micro strain of 0.04% to 0.25%, preferably 0.06% to 0.15%. If the micro strain is too large, the service life characteristics decrease, and if the micro strain is too small, the lithium ion mobility decreases.

In addition, the positive electrode active material may have an average particle diameter of primary particles of 0.05 $\mu$m to 4 pm, and preferably 0.1 $\mu$m to 2 $\mu$m. If the average particle diameter of the primary particles is too large, a rock salt phase may be formed, and thus resistance characteristics and service life characteristics may be degraded, and if the average particle diameter of the primary particles is too small, a contact area with an electrolyte may increase, and thus the deterioration may occur rapidly.

In addition, the positive electrode active material may have an average particle diameter of secondary particles of 2 $\mu$m to 25 pm, and preferably 4 $\mu$m to 18 $\mu$m. When the average particle diameter of the secondary particles satisfies the above range, it is possible to prevent the positive electrode active material particles from being broken in the rolling process or the processability from deteriorating during the preparation of slurry.

## Positive Electrode

Next, a positive electrode according to the present invention will be described.

The positive electrode includes the positive electrode active material according to the present invention. Specifically, the positive electrode includes a positive electrode collector and a positive electrode active material layer formed on the positive electrode collector, wherein the positive electrode active material layer includes the positive electrode active material according to the present invention.

In this case, since the positive electrode active material is the same as described above, detailed descriptions thereof will be omitted, and the remaining configurations will be only described in detail below.

The positive electrode collector may include a metal having high conductivity, and is not particularly limited as long as the positive electrode active material layer easily adheres thereto and there is no reactivity in the voltage range of the battery. For example, stainless steel, aluminum, nickel, titanium, fired carbon, or aluminum or stainless steel that is surface-treated with one of carbon, nickel, titanium, silver, or the like may be used as the positive electrode collector. Also, the positive electrode collector may typically have a thickness of 3 um to 500 $\mu$m, and microscopic irregularities may be formed on the surface of the collector to improve the adhesion of the positive electrode active material. For example, the positive electrode current collector may be used in various forms such as a film, a sheet, a foil, a net, a porous body, a foam, and a non-woven body.

The positive electrode active material layer may optionally include a conductive agent, a binder, and a dispersing agent, if necessary, in conjunction with the positive electrode active material.

In this case, the positive electrode active material may be included in an amount of 80 wt% to 99 wt%, for example, 85 wt% to 98.5 wt% based on a total weight of the positive electrode active material layer. When the positive electrode active material is included in an amount within the above range, excellent capacity characteristics may be obtained.

The conductive agent is used to provide conductivity to the electrode, wherein any conductive agent may be used without particular limitation as long as it has suitable electron conductivity without causing adverse chemical changes in the battery. Specific examples of the conductive agent may be graphite such as natural graphite or artificial graphite; carbon based materials such as carbon black, acetylene black, Ketjen black, channel black, furnace black, lamp black, thermal black, and carbon fibers; powder or fibers of metals such as copper, nickel, aluminum, and silver; conductive tubes such as carbon nanotubes; conductive whiskers such as zinc oxide whiskers and potassium titanate whiskers; conductive metal oxides such as titanium oxide; or conductive polymers such as polyphenylene derivatives, and any one thereof or a mixture of two or more thereof may be used. The conductive agent may be included in an amount of 0.1 wt% to 15 wt% based on the total weight of the positive electrode active material layer.

The binder improves the adhesion between the positive electrode active material particles and the adhesion between the positive electrode active material and the current collector. Specific examples of the binder may be polyvinylidene fluoride (PVDF), polyvinylidene fluoridehexafluoropropylene copolymer (PVDF-co-HFP), polyvinyl alcohol, polyacrylonitrile, polymethylmethacrylate, carboxymethyl cellulose (CMC), starch, hydroxypropyl cellulose, regenerated cellulose, polyvinylpyrrolidone, polytetrafluoroethylene, polyethylene, polypropylene, an ethylene-propylene-diene monomer(EPDM), a sulfonated EPDM, a styrene-butadiene rubber (SBR), a fluorine rubber, poly acrylic acid, and a polymer having hydrogen thereof substituted with Li, Na, or Ca, or various copolymers thereof, and any one thereof or a mixture of two or more thereof may be used. The binder may be included in an amount of 0.1 wt% to 15 wt% based on the total weight of the positive electrode active material layer.

The dispersing agent may include a water-based dispersing agent or an organic dispersing agent such as N-methyl-2-pyrrolidone.

The positive electrode may be prepared according to a typical method for preparing a positive electrode except that the above-described positive electrode active material is used. Specifically, a positive electrode slurry composition, which is prepared by dissolving or dispersing optionally the binder, the conductive agent, and the dispersing agent, if necessary, as well as the positive electrode active material in a solvent, is coated on the positive electrode collector, and the positive electrode may then be prepared by drying and rolling the coated positive electrode collector.

The solvent may be a solvent normally used in the art. The solvent may include dimethyl sulfoxide (DMSO), isopropyl alcohol, N-methylpyrrolidone (NMP), dimethyl formamide (DMF), acetone, or water, and any one thereof or a mixture of two or more thereof may be used. An amount of the solvent used may be sufficient if the solvent may dissolve or disperse the positive electrode active material, the conductive agent, the binder, and the dispersing agent in consideration of a coating thickness of the slurry and manufacturing yield, and may allow to have a viscosity that may provide excellent thickness uniformity during the subsequent coating for the preparation of the positive electrode.

Also, as another method, the positive electrode may be prepared by casting the positive electrode slurry composition on a separate support and then laminating a film separated from the support on the positive electrode collector.

## Secondary Battery

Furthermore, in the present invention, an electrochemical device including the positive electrode may be prepared. The electrochemical device may specifically be a battery or a capacitor, and, for example, may be a lithium secondary battery.

The lithium secondary battery may specifically include a positive electrode, a negative electrode disposed to face the positive electrode, a separator disposed between the positive electrode and the negative electrode, and an electrolyte. Since the positive electrode is the same as described above, detailed descriptions thereof will be omitted, and the remaining configurations will be only described in detail below.

Also, the lithium secondary battery may further optionally include a battery container accommodating an electrode assembly of the positive electrode, the negative electrode, and the separator, and a sealing member for sealing the battery container.

In the lithium secondary battery, the negative electrode includes a negative electrode collector and a negative electrode active material layer disposed on the negative electrode collector.

The negative electrode collector is not particularly limited as long as it has high conductivity without causing adverse chemical changes in the battery, and, for example, copper, stainless steel, aluminum, nickel, titanium, fired carbon, copper or stainless steel that is surface-treated with one of carbon, nickel, titanium, silver, or the like, and an aluminum-cadmium alloy may be used. Also, the negative electrode collector may typically have a thickness of 3 um to 500 $\mu$m, and as in the case of the positive electrode collector, microscopic irregularities may be formed on the surface of the negative electrode collector to improve the adhesion of a negative electrode active material. For example, the negative electrode collector may be used in various forms such as a film, a sheet, a foil, a net, a porous body, a foam, and a non-woven body.

The negative electrode active material layer optionally includes a binder and a conductive agent in addition to the negative electrode active material.

A compound capable of reversibly intercalating and deintercalating lithium may be used as the negative electrode active material. Specific examples of the negative electrode active material may be a carbonaceous material such as artificial graphite, natural graphite, graphitized carbon fibers, and amorphous carbon; a metallic compound alloyable with lithium such as Si, Al, Sn, Pb, Zn, Bi, In, Mg, Ga, Cd, a Si alloy, a Sn alloy, or an Al alloy; a metal oxide which may be doped and undoped with lithium such as $SiO_\beta$ (0 < $\beta$ < 2), $SnO_2$, vanadium oxide, and lithium vanadium oxide; or a composite including the metallic compound and the carbonaceous material such as a Si-C composite or a Sn-C composite, and any one thereof or a mixture of two or more thereof may be used. Also, a metallic lithium thin film may be used as the negative electrode active material. Furthermore, both low crystalline carbon and high crystalline carbon may be used as the carbon material. Typical examples of the low crystalline carbon may be soft carbon and hard carbon, and typical examples of the high crystalline carbon may be irregular, planar, flaky, spherical, or fibrous natural graphite or artificial graphite, Kish graphite, pyrolytic carbon, mesophase pitch-based carbon fibers, meso-carbon microbeads, mesophase pitches, and hightemperature fired carbon such as petroleum or coal tar pitch derived cokes.

The negative electrode active material may be included in an amount of 80 wt% to 99 wt% based on a total weight of the negative electrode active material layer.

The binder is a component that assists in the binding between the conductive agent, the active material, and the current collector, wherein the binder is commonly added in an amount of 0.1 wt% to 10 wt% based on the total weight of the negative electrode active material layer. Examples of the binder may be polyvinylidene fluoride (PVDF), polyvinyl alcohol, carboxymethylcellulose (CMC), starch, hydroxypropylcellulose, regenerated cellulose, polyvinylpyrrolidone, polytetrafluoroethylene, polyethylene, polypropylene, an ethylene-propylene-diene polymer (EPDM), a sulfonated-EPDM, a styrene-butadiene rubber, a fluoro rubber, and various copolymers thereof.

The conductive agent is a component for further improving conductivity of the negative electrode active material, wherein the conductive agent may be added in an amount of 10 wt% or less, for example, 5 wt% or less based on the total weight of the negative electrode active material layer. The conductive agent is not particularly limited as long as it has conductivity without causing adverse chemical changes in the battery. For example, graphite such as natural graphite or artificial graphite; carbon black such as acetylene black, Ketjen black, channel black, furnace black, lamp black, and thermal black; conductive fibers such as carbon fibers or metal fibers; fluorocarbons; metal powder such as aluminum powder or nickel powder; a conductive whisker such as a zinc oxide whisker or a potassium titanate whisker; a conductive metal oxide such as a titanium oxide; or a conductive material such as a polyphenylene derivative; and the like may be used.

The negative electrode may be prepared by coating a negative electrode slurry composition, which is prepared by dissolving or dispersing optionally the binder and the conductive agent as well as the negative electrode active material in a solvent, on the negative electrode collector and drying the coated negative electrode collector, or may be prepared by casting the negative electrode slurry composition on a separate support and then laminating a film separated from the support on the negative electrode collector.

In the lithium secondary battery, the separator separates the negative electrode and the positive electrode and provides a movement path of lithium ions, wherein any separator may be used as the separator without particular limitation as long as it is typically used in a lithium secondary battery, and particularly, a separator having high moisture-retention ability for an electrolyte as well as low resistance to the transfer of electrolyte ions may be used. Specifically, a porous polymer film, for example, a porous polymer film prepared from a polyolefin-based polymer, such as an ethylene homopolymer, a propylene homopolymer, an ethylene/butene copolymer, an ethylene/hexene copolymer, and an ethylene/methacrylate copolymer, or a laminated structure having two or more layers thereof may be used. Also, a typical porous nonwoven fabric, for example, a nonwoven fabric formed of high melting point glass fibers or polyethylene terephthalate fibers may be used. Furthermore, a coated separator including a ceramic component or a polymer material may be used to secure heat resistance or mechanical strength, and may be optionally used in a single-layered or multi-layered structure.

Also, the electrolyte used in the present invention may include an organic liquid electrolyte, an inorganic liquid electrolyte, a solid polymer electrolyte, a gel-type polymer electrolyte, a solid inorganic electrolyte, or a molten-type inorganic electrolyte which may be used in the preparation of the lithium secondary battery, but the present invention is not limited thereto.

Specifically, the electrolyte may include an organic solvent and a lithium salt.

Any organic solvent may be used as the organic solvent without particular limitation so long as it may function as a medium through which ions involved in an electrochemical reaction of the battery may move. Specifically, an esterbased solvent such as methyl acetate, ethyl acetate, $\gamma$-butyrolactone, and $\epsilon$-caprolactone; an ether-based solvent such as dibutyl ether or tetrahydrofuran; a ketone-based solvent such as cyclohexanone; an aromatic hydrocarbon-based solvent such as benzene and fluorobenzene; or a carbonate-based solvent such as dimethyl carbonate (DMC), diethyl carbonate (DEC), methylethyl carbonate (MEC), ethylmethyl carbonate (EMC), ethylene carbonate (EC), and propylene carbonate (PC); an alcohol-based solvent such as ethyl alcohol and isopropyl alcohol; nitriles such as R-CN (where R is a linear, branched, or cyclic C2-C20 hydrocarbon group and may include a doublebond, an aromatic ring or ether bond); amides such as dimethylformamide; dioxolanes such as 1,3-dioxolane; or sulfolanes may be used as the organic solvent. Among these solvents, the carbonate-based solvent may be used, and, for example, a mixture of a cyclic carbonate (e.g., ethylene carbonate or propylene carbonate) having high ionic conductivity and high dielectric constant, which may increase charge/discharge performance of the battery, and a lowviscosity linear carbonate-based compound (e.g., ethylmethyl carbonate, dimethyl carbonate, or diethyl carbonate) may be used. In this case, the performance of the electrolyte solution may be excellent when the cyclic carbonate and the chain carbonate are mixed in a volume ratio of about 1:1 to about 1:9.

The lithium salt may be used without particular limitation as long as it is a compound capable of providing lithium ions used in the lithium secondary battery. Specifically, anions of the lithium salt may be at least one selected from the group consisting of $F^-$, $Cl^-$, $Br^-$, $I^-$, $NO_3^-$, $N(CN)_2^-$, $BF_4^-$, $CF_3CF_2SO_3^-$, $(CF_3SO_2)_2N^-$, $(FSO_2)_2N^-$, $CF_3CF_2(CF_3)_2CO^-$,

$(CF_3SO_2)_2CH^-$, $(SF_5)_3C^-$, $(CF_3SO_2)_3C^-$, $CF_3(CF_2)_7SO_3^-$, $CF_3CO_2^-$, $CH_3CO_2^-$, $SCN-$, and $(CF_3CF_2SO_2)_2N^-$, and as the lithium salt, $LiPF_6$, $LiClO_4$, $LiAsF_6$, $LiBF_4$, $LiSbF_6$, $LiAlO_4$, $LiAlCl_4$, $LiCF_3SO_3$, $LiC_4F_9SO_3$, $LiN(C_2F_5SO_3)_2$, $LiN(C_2F_5SO_2)_2$, $LiN(CF_3SO_2)_2$, $LiCl$, $LiI$, $LiB(C_2O_4)_2$, or the like may be used. It is preferable to use the lithium salt in a concentration range of 0.1 M to 2.0 M. When the concentration of the lithium salt is in the above range, the electrolyte may have suitable conductivity and viscosity, thereby exhibiting excellent performance, and lithium ions may effectively move.

The lithium secondary battery including the positive electrode active material according to the present invention as describe above exhibits excellent capacity characteristics and service life characteristics, and may be useful for various fields such as portable devices such as a mobile phone, a notebook computer, and a digital camera, or electric cars.

## MODE FOR CARRYING OUT THE INVENTION

Hereinafter, the present invention will be described in detail, according to specific examples. The invention may, however, be embodied in many different forms and should not be construed as being limited to the embodiments set forth herein. Rather, these example embodiments are provided so that this description will be thorough and complete, and will fully convey the scope of the present invention to those skilled in the art.

**Examples**

**Preparation Example 1 - Preparation of Positive Electrode Active Material Precursor A**

$NiSO_4$, $CoSO_4$, and $MnSO_4$ were mixed in distilled water in an amount such that a molar ratio of nickel:cobalt:manganese was 90:5:5 to prepare a 2.4 M transition metal aqueous solution.

Then, after deionized water was put into a reactor, dissolved oxygen in the water was removed by purging the reactor with nitrogen gas to create a non-oxidizing atmosphere in the reactor. Thereafter, 7.96 M NaOH was added thereto so that a pH in the reactor was maintained at 11.9.

Thereafter, while the transition metal aqueous solution, a NaOH aqueous solution, and a $NH_4OH$ aqueous solution were added to the reactor at rates of 850 mL/hr, 510 mL/hr, and 160 mL/hr, respectively, a reaction was performed for 40 hours at a reaction temperature of 50 °C, a pH of 11.4, and a stirring speed of 600 rpm to prepare a positive electrode active material precursor represented by $Ni_{0.9}Co_{0.05}Mn_{0.05}(OH)_2$.

**Preparation Example 2 - Preparation of Positive Electrode Active Material Precursor B**

$NiSO_4$, $CoSO_4$, and $MnSO_4$ were mixed in distilled water in an amount such that a molar ratio of nickel:cobalt:manganese was 90:5:5 to prepare a 2.4 M transition metal aqueous solution.

Then, after deionized water was put into a reactor, dissolved oxygen in the water was removed by purging the reactor with nitrogen gas to create a non-oxidizing atmosphere in the reactor. Thereafter, 7.96 M NaOH was added thereto so that a pH in the reactor was maintained at 11.9.

Thereafter, while the transition metal aqueous solution, a NaOH aqueous solution, and a $NH_4OH$ aqueous solution were added to the reactor at rates of 850 mL/hr, 510 mL/hr, and 540 mL/hr, respectively, a reaction was performed for 40 hours at a reaction temperature of 50 °C, a pH of 11.4, and a stirring speed of 600 rpm to prepare a positive electrode active material precursor represented by $Ni_{0.9}Co_{0.05}Mn_{0.05}(OH)_2$.

**Example 1**

The positive electrode active material precursor A prepared in Preparation Example 1 and LiOH were mixed in an amount such that a molar ratio of Li:transition metals (Ni+Co+Mn) was 1.05:1, and the resultant mixture was fired at 770 °C for 13 hours to prepare $Li[Ni_{0.9}Co_{0.05}Mn_{0.05}]O_2$.

Then, the $Li[Ni_{0.9}Co_{0.05}Mn_{0.05}]O_2$ was washed with water and dried, then mixed with 500 ppm of boric acid, and heat-treated at 300 °C to prepare a positive electrode active material 1 coated with B.

**Example 2**

The positive electrode active material precursor A prepared in Preparation Example 1 and LiOH were mixed in an amount such that a molar ratio of Li:transition metals (Ni+Co+Mn) was 1.05:1, $Ta_2O_5$ was added thereto and mixed such that a molar ratio of transition metals:Ta was 99.75:0.25, and the resultant mixture was fired at 770 °C for 13 hours to prepare $Li[Ni_{0.9}Co_{0.05}Mn_{0.05}]_{0.9975}Ta_{0.0025}O_2$.

Then, the $Li[Ni_{0.9}Co_{0.05}Mn_{0.05}]_{0.9975}Ta_{0.0025}O_2$ was washed with water and dried, then mixed with 500 ppm of boric acid, and heat-treated at 300 °C to prepare a positive electrode active material 2 coated with B.

**Example 3**

The positive electrode active material precursor A prepared in Preparation Example 1 and LiOH were mixed in an amount such that a molar ratio of Li:transition metals (Ni+Co+Mn) was 1.05:1, $Nb_2O_3$ was added thereto and mixed such that a molar ratio of transition metals:Nb was 99.75:0.25, and the resultant mixture was fired at 770 °C for 13 hours to prepare $Li[Ni_{0.9}Co_{0.05}Mn_{0.05}]_{0.9975}Nb_{0.0025}O_2$.

Then, the $Li[Ni_{0.9}Co_{0.05}Mn_{0.05}]_{0.9975}Nb_{0.0025}O_2$ was washed with water and dried, then mixed with 500 ppm of boric acid, and heat-treated at 300 °C to prepare a positive electrode active material 3 coated with B.

**Comparative Example 1**

The positive electrode active material precursor B prepared in Preparation Example 2 and LiOH were mixed in an amount such that a molar ratio of Li:transition metals (Ni+Co+Mn) was 1.05:1, and the resultant mixture was fired at 770 °C for 13 hours to prepare $Li[Ni_{0.9}Co_{0.05}Mn_{0.05}]O_2$.

Then, the $Li[Ni_{0.9}Co_{0.05}Mn_{0.05}]O_2$ was washed with water and dried, then mixed with 500 ppm of boric acid, and heat-treated at 300 °C to prepare a positive electrode active material 4 coated with B.

**Comparative Example 2**

The positive electrode active material precursor B prepared in Preparation Example 2 and LiOH were mixed in an amount such that a molar ratio of Li:transition metals (Ni+Co+Mn) was 1.05:1, and the resultant mixture was fired at 760 °C for 13 hours to prepare $Li[Ni_{0.9}Co_{0.05}Mn_{0.05}]O_2$.

Then, the $Li[Ni_{0.9}Co_{0.05}Mn_{0.05}]O_2$ was washed with water and dried, then mixed with 500 ppm of boric acid, and heat-treated at 300 °C to prepare a positive electrode active material 5 coated with B.

**Comparative Example 3**

The positive electrode active material precursor B prepared in Preparation Example 2 and LiOH were mixed in an amount such that a molar ratio of Li:transition metals (Ni+Co+Mn) was 1.05:1, and the resultant mixture was fired at 780 °C for 13 hours to prepare $Li[Ni_{0.9}Co_{0.05}Mn_{0.05}]O_2$.

Then, the $Li[Ni_{0.9}Co_{0.05}Mn_{0.05}]O_2$ was washed with water and dried, then mixed with 500 ppm of boric acid, and heat-treated at 300 °C to prepare a positive electrode active material 6 coated with B.

**Experimental Example 1**

Each of positive electrode active materials 1 to 6 prepared according to Examples 1 to 3 and Comparative Examples 1 to 3 was cut by using an ion milling system (Hitachi, IM4000), and then the proportion of crystallites A, B, C, and D was measured by performing the scanning ion microscope analysis and EBSD analysis.

In addition, the XRD data of the positive electrode active materials prepared in Examples 1 to 3 and Comparative Examples 1 to 3 were measured by using the Empyrean equipment from Malvern panalytical Ltd., and the crystallite size and micro strain of each positive electrode active material were measured by using the Reitveld refinement method embedded in the Highscore program of Malvern panalytical, Ltd.

Measurement results are shown in Table 1 below:

[Table 1]

| | Crystallite size (nm) | Micro strain (%) | Crystallite proportion (%) | | | |
| --- | --- | --- | --- | --- | --- | --- |
| | | | Crystal lites A | Crystal lites B | Crystal lites C | Crystal lites D |
| Example 1 | 130.2 | 0.090 | 31.2 | 20.3 | 27.8 | 20.7 |
| Example 2 | 124.2 | 0.097 | 38.8 | 23.4 | 26.6 | 11.2 |
| Example 3 | 127.3 | 0.095 | 30.2 | 19.2 | 36.2 | 14.4 |
| Comparative Example 1 | 134.2 | 0.086 | 24.0 | 25.2 | 24.1 | 26.7 |

(continued)

|  | Crystallite size (nm) | Micro strain (%) | Crystallite proportion (%) | | | |
|---|---|---|---|---|---|---|
|  |  |  | Crystal lites A | Crystal lites B | Crystal lites C | Crystal lites D |
| Comparative Example 2 | 131.4 | 0.094 | 22.6 | 26.8 | 20.1 | 30.5 |
| Comparative Example 3 | 141.6 | 0.072 | 24.6 | 28.2 | 22.6 | 24.6 |

**Experimental Example 2: Evaluation of Battery Characteristics**

Each of positive electrode active materials prepared in Examples 1 to 3 and Comparative Examples 1 to 3, a conductive agent (Denka black), and a binder (PVDF) were mixed in an N-methyl-2-pyrrolidone (NMP) solvent in a weight ratio of 97.5:1:1.5 to prepare a positive electrode slurry. One surface of an aluminum current collector was coated with the positive electrode slurry, dried, and then rolled to prepare a positive electrode.

A lithium metal electrode was used as a negative electrode.

Each lithium secondary battery was prepared by preparing an electrode assembly by disposing a separator between the positive electrode and the negative electrode, disposing the electrode assembly in a battery case, and then injecting an electrolyte solution. In this case, as the electrolyte solution, an electrolyte solution was used in which 1 M $LiPF_6$ was dissolved in an organic solvent in which ethylene carbonate, ethylmethyl carbonate, and diethyl carbonate were mixed in a volume ratio of 3:3:4.

Then, each of the secondary batteries was charged at a constant current of 0.1 C to 4.2 V at 25 °C. Thereafter, each of the secondary batteries was discharged at a constant current of 0.1 C to 3 V to measure initial charge capacity and initial discharge capacity.

In addition, the charge and discharge behavior, in which each of the secondary batteries was charged at a constant current of 0.33 C to 4.2V at 45 °C and discharged at a constant current of 0.33 C to 3 V, was set as one cycle, and after this cycle was repeated 30 times, capacity retention and resistance increase rate were measured.

The capacity retention and resistance increase rate were calculated according to Equation 3 and Equation 4 below:

[Equation 3]

Capacity retention (%) = (discharge capacity after 30 cycles/discharge capacity after 1 cycle) × 100

[Equation 4]

Resistance increase rate (%) = {(resistance after 30 cycles - resistance after 1 cycle)/resistance after 1 cycle} × 100

Measurement results are shown in Table 2 below:

[Table 2]

|  | Initial capacity (mAh/g) | | Service life characteristics (30 cycles) | |
|---|---|---|---|---|
|  | Charge capacity | Discharge capacity | Capacity retention (%) | Resistance increase rate (%) |
| Example 1 | 234.2 | 215.2 | 97.0 | 92 |

(continued)

| | Initial capacity (mAh/g) | | Service life characteristics (30 cycles) | |
| --- | --- | --- | --- | --- |
| | Charge capacity | Discharge capacity | Capacity retention (%) | Resistance increase rate (%) |
| Example 2 | 232.6 | 212.8 | 98.2 | 62 |
| Example 3 | 240.1 | 219.7 | 95.6 | 104 |
| Comparative Example 1 | 230.5 | 209.8 | 94.8 | 153 |
| Comparative Example 2 | 226.5 | 204.8 | 94.0 | 208 |
| Comparative Example 3 | 224.3 | 203.1 | 93.1 | 234 |

As shown in Table 2 above, the capacity characteristics and service life characteristics of the secondary batteries using the positive electrode active materials of Examples 1 to 3 in which the proportion of the crystallites A satisfies the range of the present invention are exhibited superior to those of the secondary batteries using the positive electrode active materials of Comparative Examples 1 to 3.

**Claims**

1. A positive electrode active material for a lithium secondary battery in which the proportion of crystallites A, in which a crystallite long-axis orientation degree DoA represented by Equation 1 below is 0.5 to 1 and a crystallite c-axis orientation degree represented by an cross product value of a c-axis rotation vector Rc of a crystal lattice of a crystallite obtained by electron backscatter diffraction (EBSD) analysis and a position unit vector P' of the crystallite is 0.5 to 1, is 25% to 80% with respect to total crystallites in the cross-section of a positive electrode active material particle:

[Equation 1]

$$DoA = \frac{\lambda_1}{\lambda_1 + \lambda_2} C_D{}^2$$

wherein, in Equation 1 above,

$\lambda_1$ is the size of a long-axis vector $E_I$ of the crystallite determined from image data obtained by scanning ion microscope analysis of the cross-section of the positive electrode active material,
$\lambda_2$ is the size of a short-axis vector $E_{II}$ of the crystallite determined from image data obtained by scanning ion microscope analysis of the cross-section of the positive electrode active material, and
$C_D$ above is an inner product value of a position unit vector P' and a long-axis vector $E_I'$ of the crystallite.

2. The positive electrode active material of claim 1, wherein the scanning ion microscope analysis is performed by obtaining a scanning ion microscope image by irradiating the cross-section of the positive electrode active material with a focused ion beam, then obtaining data segmented in a crystallite unit from the scanning ion microscope image using deep learning, and calculating DoA represented by Equation 1 from the segmented data.

3. The positive electrode active material of claim 1, wherein the EBSD analysis is performed by obtaining EBSD Euler map data including position information and Euler angle information on each crystallite through EBSD measurement of the cross-section of the positive electrode active material, and calculating the c-axis rotation vector Rc (x, y, z) of the crystal lattice of the crystallite by means of Equation 2 below:

[Equation 2]

$$\begin{bmatrix} x \\ y \\ z \end{bmatrix} = R_z(\Psi)R_y(\Theta)R_x(\Phi) \begin{bmatrix} X \\ Y \\ Z \end{bmatrix}$$

$$= \begin{bmatrix} \cos\Psi & -\sin\Psi & 0 \\ \sin\Psi & \cos\Psi & 0 \\ 0 & 0 & 1 \end{bmatrix} \begin{bmatrix} \cos\Theta & 0 & \sin\Theta \\ 0 & 1 & 0 \\ -\sin\Theta & 0 & \cos\Theta \end{bmatrix} \begin{bmatrix} 1 & 0 & 0 \\ 0 & \cos\Phi & -\sin\Phi \\ 0 & \sin\Phi & \cos\Phi \end{bmatrix} \begin{bmatrix} X \\ Y \\ Z \end{bmatrix}$$

$$= \begin{bmatrix} \cos\Theta\cos\Psi & -\cos\Phi\sin\Psi+\sin\Phi\sin\Theta\cos\Psi & \sin\Phi\sin\Psi+\cos\Phi\sin\Theta\cos\Psi \\ \cos\Theta\sin\Psi & \cos\Phi\cos\Psi+\sin\Phi\sin\Theta\sin\Psi & -\sin\Phi\cos\Psi+\cos\Phi\sin\Theta\sin\Psi \\ -\sin\Theta & \sin\Phi\cos\Theta & \cos\Phi\cos\Theta \end{bmatrix} \begin{bmatrix} X \\ Y \\ Z \end{bmatrix}$$

wherein, in Equation 2 above,
[X, Y, Z] is (0, 0, 1), and $\Phi$, $\Theta$, and $\Psi$ above each represent Euler angle obtained from Euler map data.

4. The positive electrode active material of claim 1, further comprising:

crystallites B in which the crystallite c-axis orientation degree is 0.5 to 1 and the DoA is less than 0.5;
crystallites C in which the crystallite c-axis orientation degree is less than 0.5 and the DoA is 0.5 to 1; and
crystallites D in which the crystallite c-axis orientation degree is less than 0.5 and the DoA is less than 0.5.

5. The positive electrode active material of claim 4, wherein the proportion of the sum of the crystallites A and the crystallites C in the total crystallites in the cross-section of the positive electrode active material particle is 50% to 90%.

6. The positive electrode active material of claim 4, wherein the proportion of the crystallites A in the total crystallites in the cross-section of the positive electrode active material particle is 25% to 70%, the proportion of the crystallites B is 5% to 30%, the proportion of the crystallites C is 20% to 70%, and the proportion of the crystallites D is 5% to 30%.

7. The positive electrode active material of claim 1, wherein the positive electrode active material has a crystallite size of 100 nm to 200 nm.

8. The positive electrode active material of claim 1, wherein the positive electrode active material has a micro strain of 0.04% to 0.25%.

9. The positive electrode active material of claim 1, wherein the positive electrode active material has an average particle diameter of primary particles of 0.05 um to 8 $\mu$m.

10. The positive electrode active material of claim 1, wherein the positive electrode active material has an average particle diameter of secondary particles of 2 um to 25 $\mu$m.

11. The positive electrode active material of claim 1, wherein the positive electrode active material is a lithium composite transition metal oxide represented by Formula 1 below:

[Formula 1]  $Li_x[Ni_aCo_bM^1_cM^2_d]O_{2-y}A_y$

wherein, in Formula 1 above,

$M^1$ above is at least one element selected from the group consisting of Mn and Al,
$M^2$ above is at least one element selected from the group consisting of W, Cu, Fe, V, Cr, Ti, Zr, Zn, Al, Ta, Y, In, La, Sr, Ga, Sc, Gd, Sm, Ca, Ce, Nb, Mg, B, and Mo,
A above is at least one element selected from the group consisting of F, Cl, Br, I, At, and S, and
$0.98 \leq x \leq 1.20$, $0<a<1$, $0<b<1$, $0<c<1$, $0 \leq d \leq 0.2$, and $0 \leq y \leq 0.2$.

**12.** A positive electrode comprising the positive electrode active material of any one of claims 1 to 11.

**13.** A lithium secondary battery comprising the positive electrode of claim 12.

[FIG. 1]

[FIG. 2]

**DL Boundary detection**

ML segmentation

565

[FIG. 3]

[FIG. 4]

[FIG. 5]

## INTERNATIONAL SEARCH REPORT

| International application No. |
| --- |
| **PCT/KR2022/003972** |

**A.    CLASSIFICATION OF SUBJECT MATTER**

**H01M 4/525**(2010.01)i; **H01M 4/505**(2010.01)i; **H01M 4/36**(2006.01)i; **H01M 4/62**(2006.01)i; **H01M 10/052**(2010.01)i; **H01M 4/02**(2006.01)i

According to International Patent Classification (IPC) or to both national classification and IPC

**B.    FIELDS SEARCHED**

Minimum documentation searched (classification system followed by classification symbols)

H01M 4/525(2010.01); H01M 10/052(2010.01); H01M 4/485(2010.01); H01M 4/58(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 양극활물질(positive electrode active material), 결정립(crystalline), 배향도 (orientation)

**C.    DOCUMENTS CONSIDERED TO BE RELEVANT**

| Category* | Citation of document, with indication, where appropriate, of the relevant passages | Relevant to claim No. |
| --- | --- | --- |
| A | KR 10-2017-0115939 A (IUCF-HYU (INDUSTRY-UNIVERSITY COOPERATION FOUNDATION HANYANG UNIVERSITY)) 18 October 2017 (2017-10-18) See claims 1, 6 and 9; and paragraphs [0061] and [0224]-[0228]. | 1-13 |
| DA | KR 10-1611784 B1 (IUCF-HYU (INDUSTRY-UNIVERSITY COOPERATION FOUNDATION HANYANG UNIVERSITY) et al.) 14 April 2016 (2016-04-14) See claims 1-6. | 1-13 |
| A | KR 10-2021-0007808 A (IUCF-HYU (INDUSTRY-UNIVERSITY COOPERATION FOUNDATION HANYANG UNIVERSITY)) 20 January 2021 (2021-01-20) See entire document. | 1-13 |
| A | KR 10-2020-0084565 A (LG CHEM, LTD.) 13 July 2020 (2020-07-13) See entire document. | 1-13 |
| A | KR 10-2018-0077081 A (LG CHEM, LTD.) 06 July 2018 (2018-07-06) See entire document. | 1-13 |

☐ Further documents are listed in the continuation of Box C.       ☑ See patent family annex.

| | Special categories of cited documents: | | |
| --- | --- | --- | --- |
| "A" | document defining the general state of the art which is not considered to be of particular relevance | "T" | later document published after the international filing date or priority date and not in conflict with the application but cited to understand the principle or theory underlying the invention |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **22 June 2022** | **22 June 2022** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.

**PCT/KR2022/003972**

| Patent document cited in search report | | | Publication date (day/month/year) | Patent family member(s) | | | Publication date (day/month/year) |
|---|---|---|---|---|---|---|---|
| KR | 10-2017-0115939 | A | 18 October 2017 | CN | 108883949 | A | 23 November 2018 |
| | | | | CN | 108883949 | B | 26 February 2021 |
| | | | | CN | 109071265 | A | 21 December 2018 |
| | | | | CN | 109415224 | A | 01 March 2019 |
| | | | | EP | 3441364 | A1 | 13 February 2019 |
| | | | | EP | 3441364 | A4 | 11 December 2019 |
| | | | | EP | 3441365 | A1 | 13 February 2019 |
| | | | | EP | 3441365 | A4 | 11 December 2019 |
| | | | | EP | 3441366 | A2 | 13 February 2019 |
| | | | | EP | 3441366 | A4 | 22 January 2020 |
| | | | | KR | 10-2017-0115938 | A | 18 October 2017 |
| | | | | KR | 10-2017-0116569 | A | 19 October 2017 |
| | | | | US | 10797318 | B2 | 06 October 2020 |
| | | | | US | 10879532 | B2 | 29 December 2020 |
| | | | | US | 2019-0044140 | A1 | 07 February 2019 |
| | | | | US | 2019-0044141 | A1 | 07 February 2019 |
| | | | | US | 2019-0044142 | A1 | 07 February 2019 |
| | | | | WO | 2017-175977 | A1 | 12 October 2017 |
| | | | | WO | 2017-175978 | A1 | 12 October 2017 |
| | | | | WO | 2017-175979 | A2 | 12 October 2017 |
| | | | | WO | 2017-175979 | A3 | 02 August 2018 |
| KR | 10-1611784 | B1 | 14 April 2016 | CN | 104521039 | A | 15 April 2015 |
| | | | | CN | 104521039 | B | 29 March 2017 |
| | | | | CN | 105576198 | A | 11 May 2016 |
| | | | | CN | 105576198 | B | 29 January 2019 |
| | | | | CN | 107093740 | A | 25 August 2017 |
| | | | | CN | 107093740 | B | 01 December 2020 |
| | | | | CN | 107293689 | A | 24 October 2017 |
| | | | | CN | 107293689 | B | 23 April 2021 |
| | | | | EP | 2882013 | A1 | 10 June 2015 |
| | | | | EP | 2882013 | A4 | 11 November 2015 |
| | | | | EP | 2882013 | B1 | 12 January 2022 |
| | | | | EP | 3016184 | A2 | 04 May 2016 |
| | | | | EP | 3016184 | A3 | 10 August 2016 |
| | | | | EP | 3016184 | B1 | 10 April 2019 |
| | | | | EP | 3236517 | A1 | 25 October 2017 |
| | | | | EP | 3236517 | B1 | 04 March 2020 |
| | | | | KR | 10-1510940 | B1 | 10 April 2015 |
| | | | | KR | 10-1720042 | B1 | 30 March 2017 |
| | | | | KR | 10-1802568 | B1 | 30 November 2017 |
| | | | | KR | 10-1849719 | B1 | 19 April 2018 |
| | | | | KR | 10-2013-0138073 | A | 18 December 2013 |
| | | | | KR | 10-2013-0138147 | A | 18 December 2013 |
| | | | | KR | 10-2016-0043531 | A | 21 April 2016 |
| | | | | KR | 10-2016-0052936 | A | 13 May 2016 |
| | | | | KR | 10-2017-0017969 | A | 15 February 2017 |
| | | | | US | 10224541 | B2 | 05 March 2019 |
| | | | | US | 10283757 | B2 | 07 May 2019 |
| | | | | US | 10950856 | B2 | 16 March 2021 |
| | | | | US | 2014-0158932 | A1 | 12 June 2014 |

Form PCT/ISA/210 (patent family annex) (July 2019)

**INTERNATIONAL SEARCH REPORT**
Information on patent family members

International application No.

**PCT/KR2022/003972**

| Patent document cited in search report | | | Publication date (day/month/year) | Patent family member(s) | | | Publication date (day/month/year) |
|---|---|---|---|---|---|---|---|
| | | | | US | 2016-0049647 | A1 | 18 February 2016 |
| | | | | US | 2016-0218350 | A1 | 28 July 2016 |
| | | | | US | 2019-0148721 | A1 | 16 May 2019 |
| | | | | US | 9337487 | B2 | 10 May 2016 |
| | | | | WO | 2013-183974 | A1 | 12 December 2013 |
| KR | 10-2021-0007808 | A | 20 January 2021 | CN | 114365308 | A | 15 April 2022 |
| | | | | EP | 3989316 | A1 | 27 April 2022 |
| | | | | KR | 10-2334909 | B1 | 03 December 2021 |
| | | | | WO | 2021-006520 | A1 | 14 January 2021 |
| KR | 10-2020-0084565 | A | 13 July 2020 | None | | | |
| KR | 10-2018-0077081 | A | 06 July 2018 | CN | 108336326 | A | 27 July 2018 |
| | | | | CN | 110352518 | A | 18 October 2019 |
| | | | | JP | 2020-507894 | A | 12 March 2020 |
| | | | | JP | 2021-168306 | A | 21 October 2021 |
| | | | | JP | 6945879 | B2 | 06 October 2021 |
| | | | | KR | 10-2018-0077018 | A | 06 July 2018 |
| | | | | KR | 10-2024744 | B1 | 25 September 2019 |
| | | | | KR | 10-2117621 | B1 | 02 June 2020 |
| | | | | US | 2018-0183046 | A1 | 28 June 2018 |
| | | | | WO | 2018-143753 | A1 | 09 August 2018 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020210036940

- KR 101611784